# EUROPÄISCHE PATENTSCHRIFT

(11) **EP 1 809 233 B1**
(45) Veröffentlichungstag und Bekanntmachung des Hinweises auf die Patenterteilung: **23.12.2015**
(21) Anmeldenummer: 05806791.9
(22) Anmeldetag: 11.11.2005
(51) Int. Cl.: A61K 8/24, A61K 8/65, A61Q 11/00, A61K 8/73, A61K 6/033, A61K 8/19, A61K 6/027

(54) **INDUZIERTE REMINERALISATION VON HUMANEM ZAHNSCHMELZ**
INDUCED REMINERALISATION OF HUMAN DENTAL ENAMEL
REMINERALISATION INDUITE D'EMAIL DENTAIRE CHEZ L'HOMME

(30) Priorität: 11.11.2004 DE 102004054584
(43) Veröffentlichungstag der Anmeldung: 25.07.2007
(73) Patentinhaber: Max-Planck-Gesellschaft zur Förderung der Wissenschaften e.V., 80539 München (DE)
(72) Erfinder: BUSCH, Susanne, 01326 Dresden (DE); KNIEP, Rüdiger, 40764 Langenfeld (DE)
(74) Vertreter: Weickmann & Weickmann
(86) Internationale Anmeldenummer: PCT/EP2005/012101
(87) Internationale Veröffentlichungsnummer: WO 2006/050966

(56) Entgegenhaltungen:
- WO-A-98/10736
- WO-A-03/099234
- DE-A- 2 131 666
- DE-A- 2 350 548
- DE-A- 3 303 937

## Beschreibung

Die vorliegende Anmeldung betrifft ein Kit, welches für die induzierte Remineralisation von humanem Zahnschmelz und insbesondere den Aufbau von Apatit auf Zahnmaterial geeignet ist und welches weiterhin für die induzierte Remineralisation von humanem Dentin und insbesondere den Aufbau von Dentin auf Zahnmaterial geeignet ist.

Zähne sind Verbundstoffe aus Apatit und Proteinen. Es handelt sich bei ihnen um sehr harte Biomaterialien auf der Basis von Calcium und Phosphat. Der Zahnschmelz, die äußere Schicht der Zahnkrone ist der härteste Teil des Zahns und enthält keine lebenden Zellen. Zahnschmelz besteht aus anorganischen Kristallen, welche typische hoch orientierte Anordnungen aufweisen. Zahnschmelz ist ein Gewebe, das, sobald es einmal gebildet ist, lebenslang nahezu unverändert bleibt, da die Zellen, welche beim Aufbau der Zähne beteiligt sind, absterben, sobald die Zahnbildung abgeschlossen ist. Ein fertiger Zahnschmelz besteht aus etwa 95 Gew.-% Apatit, etwa 3 Gew.-% Proteinen und Lipiden und etwa 2 Gew.-% Wasser.

Dentin ist die Bezeichnung für eine dem Knochen verwandte Hartsubstanz, die den Kern des Zahnes bei Säugetieren und dem Menschen bildet. Dentin besteht zu ca. 30 % aus einer zellfreien organischen Grundsubstanz, insbesondere Glycoproteinen, in die Kollagenfasern eingelagert sind. Die anorganischen Bestandteile sind vor allem Hydroxylapatit, Fluorapatit und geringe Mengen an Carbonaten, Magnesium und Spurenelementen.

Um Schädigungen von Zähnen, insbesondere durch Karies zu vermeiden oder zu reparieren, wurde seit langem versucht, remineralisierende Systeme einzusetzen. Dabei wurde zunächst versucht, durch Aufbringen von Calciumphosphatverbindungen die Beschaffenheit der Zähne zu verbessern. Solche Einkomponentensysteme, bei denen versucht wird, bereits vorgefertigtes Zahnmaterial, beispielsweise Apatit, Hydroxyapatit oder andere Calciumphosphatverbindungen auf die Zähne aufzubringen, sind unter anderem in EP 0 666 730 B1 oder WO 01/95863 beschrieben. Das Problem solcher Systeme besteht darin, dass das Behandeln von Zahnmaterial mit Calciumphosphatverbindungen nicht zu einem Aufwachsen von strukturell dem Zahnmaterial ähnlichem Apatit führt, sondern vielmehr zu einer bloßen Anlagerung von Apatitkristallen auf dem Zahnmaterial, wobei die Apatitkristalle eine vom Zahnmaterial völlig unterschiedliche Morphologie aufweisen. Somit wird keine Festigung des Zahnschmelzes oder dauerhafte Füllung von Läsionen bewirkt, da die angelagerten Apatitkristalle keine ausreichende Ähnlichkeit und Haftung zum natürlichen Zahnmaterial aufweisen.

Weiterhin wurde versucht mit Zweikomponentensystemen eine Remineralisierung von Zähnen zu erhalten, wobei die Systeme üblicherweise eine Calciumphase sowie eine Phosphatphase umfassen. Zweikomponentensysteme sind beispielsweise in WO 98/10736 und DE 33 03 937 A1 beschrieben. Nachteilig bei den dort beschriebenen Vorgehensweisen ist, dass die in WO98/10736 beschriebene Methode Calcium- und Phosphatlösungen vor der Anwendung vereinigt, sodass sich eine metastabile Lösung bildet, aus der auf dem Zahn Apatit auskristallisieren soll. Die Methode erlaubt keine lokalisierte Behandlung am Zahn, da das Reagenz als Mundspülung oder Gel eingesetzt wird, das mit der Zahnbürste einmassiert wird. Weiterhin wird die Kompositnatur des nativen Schmelzes nicht berücksichtigt, da im System keine organische Komponente enthalten ist. Die Bildung zahnschmelzähnlicher Kristallite ist demnach unwahrscheinlich. DE 33 03 937 beschreibt ein Verfahren, bei dem Calcium- und Phosphationen getrennt nacheinander auf den Zahn aufgebracht werden, indem dieser in eine Kappe getaucht wird, welche die entsprechenden Ionen in einer Gelatinematrix enthält. Bei einer empfohlenen Einwirkungszeit von nur zwei Minuten ist nicht zu erwarten, dass sich wirklich größere Mengen Apatit auf der Zahnoberfläche bilden können. Es ist nicht mit Bildmaterial belegt, dass die neugebildete Apatitschicht schmelzähnliche Strukturen aufweist.

In weiteren Arbeiten (S. Busch et al., Eur. J. Inorg. Chem. (1999), 1643-1653; S. Busch et al., Chem. Mater. 13 (2001), 3260-3271; S. Busch, Zahnärztliche Mitteilungen 91, Nr. 10 (2001), 34-38; R. Kniep et al., Angew. Chem. 108, Nr. 22 (1996), 2787-2791) wurde die biomimetische Morphogenese von Fluorapatit-Gelatinkompositen untersucht. Dabei wurde biomimetisches Wachstum und Selbstorganisation von Fluorapatit-Aggregaten durch Diffusion in denaturierten Collagen-Matrizes beobachtet. Die Grundlagen der Fluorapatitbildung in Gelatine-Gelen wurde dabei mittels Doppeldiffusionsversuchen von Calcium- und Phosphatlösungen in einem U-Rohr untersucht. Diese Arbeiten beschreiben die Bildung von Fluorapatitkügelchen innerhalb des verwendeten Gels.

WO 03/099234 A1 beschreibt ein Verfahren zum Aufwachsen von Apatit auf Zahnmaterial, umfassend die Schritte:
(i) Auftragen eines ersten Gels, welches Gelatine sowie Phosphationen umfasst,
(ii) Auftragen eines zweiten Gels, wobei mit diesem zweiten Gel die erste Gelschicht abgedeckt wird und
(iii) Auftragen eines Calciumionen enthaltenden Mediums,
wobei ein Aufbau von Apatit an der Oberfläche des Zahnmaterials bewirkt wird.

Mit diesem Verfahren konnten bereits gute Ergebnisse erhalten werden. Es war jedoch wünschenswert, dieses Verfahren, insbesondere im Hinblick auf die Wachstumsgeschwindigkeit der Apatitschicht weiter zu verbessern.

Eine Aufgabe der vorliegenden Anmeldung war es deshalb, ein Kit bereitzustellen, mit dem Defekte an Zahnmaterial durch Remineralisation mit hoher Wachstumsrate ausgebessert werden können.

Diese Aufgabe wird erfindungsgemäß gelöst durch ein Kit, umfassend
(i) ein alkalisches Medium zur Vorbehandlung eines Zahnmaterials,
(ii) ein erstes Gel, welches Gelatine sowie Phosphationen umfasst, wobei die Erweichungstemperatur des ersten Gels im Bereich von 38 bis 45 °C liegt und wobei das erste Gel einen pH-Wert von 2,0 bis 6,0 aufweist, zum Auftragen auf das mit dem alkalischen Medium vorbehandelte Zahnmaterial, sowie
(iii) ein zweites Gel, welches frei von Phosphationen ist, zum Abdecken der ersten Gelschicht, wobei die Erweichungstemperatur des zweiten Gels im Bereich von 38 bis 45 °C liegt, und
(iva) ein Calciumionen enthaltendes Medium, zum Auftragen auf die zweite Gelschicht
   oder
(ivb) ein Calciumionen enthaltendes zweites Gel zum Abdecken der ersten Gelschicht anstelle des Gels (iii), wobei die Erweichungstemperatur des zweiten Gels im Bereich von 38 bis 45 °C liegt.

Dieser Kit umfasst somit die Komponenten (i), (ii), (iii) und (iva) oder die Komponenten (i), (ii) und (ivb).

Das Kit ist insbesondere zum Aufwachsen von Apatit oder/und Dentin auf Zahnmaterial geeignet.

Überraschenderweise wurde festgestellt, dass durch Vorbehandlung des Zahnmaterials mit einem alkalischen Medium eine deutliche Erhöhung der Wachstumsgeschwindigkeit von Fluorapatit auf Zahnproben, insbesondere humanen Zahnproben, erzielt werden kann. Insbesondere konnten Wachstumsraten von 1 bis 5 µm/Tag, insbesondere von 3 bis 5 µm/Tag erreicht werden.

Erfindungsgemäß bevorzugt wird als alkalisches Medium eine alkalische Lösung, insbesondere eine wässrige alkalische Lösung, oder ein alkalisches Gel eingesetzt. Das alkalische Medium hat dabei bevorzugt einen pH-Wert von 7,1 bis 14, insbesondere von mindestens 7,3, mehr bevorzugt von mindestens 7,5 und am meisten bevorzugt von mindestens 8 und bevorzugt bis zu 10, mehr bevorzugt bis zu 9. Besonders vorteilhaft wird als alkalisches Medium eine Zusammensetzung eingesetzt, die im Mundraum von Menschen verträglich ist, beispielsweise eine 0,05 bis 1N NaOH-Lösung. Es hat sich herausgestellt, dass das alkalische Medium, und insbesondere Natronlauge, weiterhin vorteilhafterweise bereits Calciumionen enthalten kann, beispielsweise 10 bis 50 % einer 0,1 bis 0,3N CaCl₂-Lösung.

Mit dem erfindungsgemäßen Kit ist es möglich, ein wirkliches Aufwachsen von Zahnschmelz-ähnlichem Material zu erzielen. Ein wesentlicher Vorteil besteht darin, dass eine hohe Ordnung kleiner Apatitnadeln erhalten wird, die strukturell eine große Ähnlichkeit zu nativem Zahnschmelz aufweisen. Bei entsprechender Substratorientierung ist praktisch kein Unterschied zwischen aufgewachsenem Apatit und ursprünglichem Zahnmaterial zu erkennen.

Mit dem erfindungsgemäßen Kit ist es weiterhin möglich, Dentin zu remineralisieren. Dabei kann ein Aufwachsen von Dentinschichten oder Dentinähnlicher Schichten auf Zahnmaterial erzielt werden. Die aufgewachsene Schicht entspricht in Kristallitgröße und Kristallitordnung natürlichem Dentin.

Weitere Vorteile der Erfindung sind, dass von einem echten Aufwachsen der Fluorapatitkristallite auf dem Zahnsubstrat ausgegangen werden kann. Die Vickers-Härte dieser neuen Schicht entspricht dem natürlichen Schmelz bzw. natürlichem Dentin. Die Durchführung der einzelnen Schritte ist so einfach, dass die Remineralisation von Zahnschmelz oder die Remineralisation von Dentin im Prinzip vom Patienten selbst durchgeführt werden kann. Das Gel kann lokal auf die geschädigten Stellen aufgetragen werden und verfestigt sich dort. Da das erwärmte Gel sehr schnell abkühlt, sind kaum Wartezeiten zwischen den einzelnen Schritten nötig.

Da die Erweichungstemperatur des Gels etwas über der normalen Körpertemperatur liegt (38 bis 42 °C), wird ein Schmelzen des Gels während der Einwirkungsdauer verhindert. Damit kann eine unkontrollierte Mineralisation vermieden werden.

Mit dem erfindungsgemäßen Kit wird Apatit auf Zahnmaterial aufgewachsen, indem
(i) ein alkalisches Medium aufgetragen wird, und
(ii) ein erstes Gel, welches Gelatine sowie Phosphationen umfasst, auf das mit dem alkalischen Medium vorbehandelte Zahnmaterial aufgetragen wird.

Weiterhin wird (iii) eine zweites Gel, welches frei von Phosphationen ist, aufgetragen, wobei mit diesem zweiten Gel die erste Gelschicht abgedeckt wird, und (iva) ein Calciumionen enthaltendes Medium wird auf die zweite Gelschicht aufgetragen. Bei Verwendung eines Calciumionen enthaltenden Gels (ivb) als Calciumionen enthaltendes Medium kann dieses Gel direkt (also ohne Behandlung mit dem Gel (iii)) auf das erste Gel aufgebracht werden.

Erfindungsgemäß wurde weiterhin festgestellt, dass durch Verwendung eines ersten Gels, welches weiterhin mindestens eine Calciumphosphatverbindung enthält, ebenfalls eine Verbesserung der Wachstumsgeschwindigkeit von Fluorapatit erzielt werden kann. Geeignete Calciumphosphatverbindungen sind beispielsweise Fluorapatit, Monetit, Brushit, amorphes Calciumphosphat, Hydroxylapatit etc. Die Calciumphosphatverbindung wird bevorzugt in Form von Partikeln und am meisten bevorzugt in Form von sphärischen oder überwiegend sphärischen Partikeln eingesetzt. Die Größe der Partikel beträgt dabei vorzugsweise 5 bis 50 µm, insbesondere 10 bis 20 µm. Geeigneterweise wird dem ersten Gel 5 bis 30 Gew.-% an Calciumphosphatverbindungen zugesetzt. In einer besonders bevorzugten Ausführungsform umfasst das erste Gel 5 bis 30 Gew.-% überwiegend sphärischer Fluorapatitpartikel mit einer Größe von 5 bis 50 µm.

Durch Zugabe von Fluoridionen zum phosphathaltigen Gel kann die Resistenz der Schicht gegenüber Säuren erhöht werden.

Mit dem erfindungsgemäßen Kit ist es möglich durch induzierte Remineralisation Zahnschmelzdefekte zu regenerieren. Durch den Einsatz eines zweischichtigen Gels, welches bei Körpertemperatur fest ist und lokal auf die betroffene Stelle am Zahn aufgebracht werden kann sowie durch die Verwendung einer Mundspülung als Calciumionen enthaltenden Medium werden Mineralisationsbedingungen geschaffen, welche die Bildung einer Zahnschmelz-ähnlichen Substanz bewirken, die direkt auf dem Zahn aufwächst. Bei der bisher veröffentlichten Doppeldiffusionsmethode wurde lediglich gezeigt, dass Fluorapatit, der durch Gegenstromdiffusion von Calcium- und Phosphationen in einem Gelatinegel entsteht, kugelige Aggregate bildet, deren organischer Gewichtsanteil dem von reifem, menschlichem Zahnschmelz entspricht. Die Doppeldiffusionsmethode hat aber weder eine Möglichkeit eröffnet, am Menschen die Remineralisation von Zahnschmelz zu ermöglichen noch diese Möglichkeit in irgendeiner Weise impliziert. Der bei der Doppeldiffusionsmethode eingesetzte Versuchsaufbau bewirkt die Bildung von kleinen Kügelchen und erlaubt nicht das Aufwachsen von gleichmäßigen Schichten von Apatitmaterial auf einem Substrat. Dies ist erst durch das erfindungsgemäße Kit möglich.

Weiterhin ist es möglich, durch induzierte Remineralisation von Dentin auch Zahnkerndefekte zu regenerieren. Dabei werden Dentinschichten gebildet, die direkt auf dem Zahnmaterial anwachsen.

Das erfindungsgemäße Kit lässt sich insbesondere beim Menschen anwenden. Dabei können beispielsweise kleinere kariöse Defekte durch induzierte Remineralisation ausgeheilt oder empfindliche Stellen am Zahn mit einer schützenden Apatitschicht bedeckt werden. Die Vorgehensweise zur Behandlung ist dabei bevorzugt wie folgt: Die kariöse Stelle wird zunächst mit einem alkalischen Medium, z.B. mit Natronlauge, behandelt und dann mit einer dünnen Schicht des etwa 50 °C warmen phosphathaltigen Gels bestrichen oder mit einer geeigneten Spritze aufgetragen, die gewärmt werden kann. Das Gel erstarrt sofort auf der Zahnoberfläche und wird nach der gleichen Methode mit dem Schutzgel bzw. einem Calciumionen enthaltenden Mittel abgedeckt. 1- bis 3-mal am Tag wird dann gegebenenfalls mit einer Calciumlösung eine etwa 10-minütige Mundspülung durchgeführt. Anstelle der Mundspülung kann auch ein Auftragen eines Calciumionen enthaltenden Gels vorgenommen werden. Bevorzugt ist z.B. ein 0,1 bis 0,5 N Calcium-Gel, durch welches die Applikation weiter vereinfacht wird. Zwischen den Spülungen wird der Zahn mit einer passenden Kappe abgedeckt, die aus Plastik oder Metall sein kann, sodass der Patient nicht behindert ist und die Remineralisation ungestört stattfinden kann. Wenn viele Zähne betroffen sind, kann auch die ganze Zahnreihe mit einer Schiene geschützt werden, wie sie z.B. gegen Zähneknirschen eingesetzt wird. Alle zwei Tage wird das Gel gewechselt, zu dieser Gelegenheit wird der betroffene Zahn gereinigt und desinfiziert.

Bei Verwendung des erfindungsgemäßen Kits wird auf das Zahnmaterial ein erstes Gel aufgebracht. Dieses Gel enthält Gelatine sowie Phosphationen und gegebenenfalls weitere Bestandteile, insbesondere Calciumphosphate, wie oben erläutert. Der Gehalt an Gelatine im ersten Gel beträgt bevorzugt von mindestens 15 Gew.-%, mehr bevorzugt von 25 Gew.-% bis zu 40 Gew.-%, mehr bevorzugt bis zu 30 Gew.-%. Der Gelatine kommt insbesondere eine Funktion bei der Ausbildung der Morphologie des gebildeten Apatits zu. Es wurde überraschenderweise festgestellt, dass bei der Verwendung von Gelatine ein Apatitmaterial an der Oberfläche von Zahnmaterial abgeschieden wird, welches eine große Ähnlichkeit mit nativem Zahnschmelz bzw. Dentin aufweist. Bei Verwendung anderer organischer Matrizes wurden hingegen andere Morphologien der Apatitkristallisate beobachtet, sodass es nicht zu einem Aufbau von Apatit an der Oberfläche des Zahnmaterials, wie erfindungsgemäß angestrebt, kommt.

Gelatine ist ein Polypeptid, welches insbesondere durch Hydrolyse des in Haut und Knochen von Tieren enthaltenen Collagens gewonnen werden kann. Gelatine weist üblicherweise ein Molekulargewicht von 15.000 bis über 250.000 g/mol auf und kann aus Collagen unter sauren oder alkalischen Bedingungen gewonnen werden. Erfindungsgemäß bevorzugt werden folgende Gelatinen eingesetzt: Sauer hydrolysierte Gelatinesorten (Typ A), z.B. hergestellt aus Schweineschwarte oder Kalbshaut mit hohem BloomWert, z.B. 250 bis 350 Bloom (unter dem Bloomwert versteht man eine Kenngröße, welche die Gelfestigkeit kennzeichnet, im Allgemeinen gilt, je höher der Bloomwert, umso höher der Anteil langkettiger Moleküle in der Gelatine und um so höher die Gelfestigkeit).

Neben Gelatine, welche zur Ausbildung der gewünschten Morphologie des Apatits und den Aufbau an der Oberfläche des Zahnmaterials enthalten ist, umfasst das erste Gel weiterhin Phosphationen. Diese Phosphationen stellen einen Grundbestandteil des aus Calciumphosphat aufgebauten Apatits dar. Die Konzentration der Phosphationen im ersten Gel beträgt bevorzugt mindestens 0,01 mol/l, mehr bevorzugt mindestens 0,05 mol/l und bis zu 0,5 mol/l, mehr bevorzugt bis zu 0,2 mol/l und insbesondere 0,08 mol/l.

Das erste Gel weist eine Erweichungstemperatur auf, die über der normalen Körpertemperatur liegt, sodass das Gel bei Körpertemperatur fest ist. Die Erweichungstemperatur des ersten Gels liegt im Bereich von 38 bis 45 °C, mehr bevorzugt von 38 bis 42 °C. Das erste Gel wird bevorzugt in erwärmter Form, beispielsweise auf 45 bis 55 °C erwärmt aufgetragen. Nach dem Auftragen kühlt das Gel ab und wird fest.

Bei Verwendung des erfindungsgemäßen Kits kann in einem weiteren Schritt ein zweites Gel, ein sogenanntes Schutzgel aufgetragen werden. Mit diesem zweiten Gel wird insbesondere die erste Gelschicht abgedeckt. Das Schutzgel, welches als Geldeckschicht fungiert, bewirkt überraschenderweise, dass die Mineralisation, also die Bildung von Apatit, überwiegend oder ausschließlich an der Zahnoberfläche und nicht an der Grenzschicht Gel-Flüssigkeit stattfindet. Durch den zweischichtigen Gelaufbau, der mit dem erfindungsgemäßen Kit erzielt wird, kommt es zu einem Aufbau bzw. Aufwachsen von Apatit auf dem Zahnmaterial und nicht zu einer Kristallisation oder Ausbildung von Apatitkugeln innerhalb des Gels, wie es im Stand der Technik beschrieben wird. Durch den zweischichtigen Aufbau ist somit eine praktikable und technisch sinnvolle Remineralisation der Zähne möglich.

Der pH-Wert und die Gelkonzentrationen des zweiten Gels entsprechen typischerweise denen, die hierin für das erste Gel angegeben sind. Auch das zweite Gel weist eine Erweichungstemperatur von 38 bis 45 °C, insbesondere von 38 bis 42 °C auf und wird bevorzugt auf 45 bis 55 °C erwärmt aufgebracht.

Bei Verwendung des erfindungsgemäßen Kits wird in einem dritten Schritt oder anstelle des Schutzgels (insbesondere bei Verwendung eines Calciumionen enthaltenden Gels) schließlich ein Calciumionen enthaltendes Medium aufgebracht. Das Calciumionen enthaltende Medium stellt den weiteren zur Bildung von Apatit benötigten Grundbaustoff, nämlich Calciumionen bereit. Diese Calciumionen diffundieren durch das Schutzgel und die erste Gelschicht bis zur Oberfläche des Zahnmaterials und werden dort als Apatit abgeschieden. Die Konzentration der Calciumionen im Calciumionen enthaltenden Medium beträgt vorzugsweise mindestens 0,01 mol/l, mehr bevorzugt mindestens 0,05 mol/l und bis zu 0,5 mol/l, mehr bevorzugt bis zu 0,2 mol/l und insbesondere 0,13 mol/l.

Es wurde festgestellt, dass mit dem erfindungsgemäßen Kit eine gleichmäßige Schicht paralleler oder strahlig gewachsener Apatitkristallite gebildet werden kann. Weiterhin zeigt diese Schicht keinen oder nur einen Submikrometer großen Randspalt zum nativen Zahnmaterial auf. Die Wachstumsrichtung der Apatitkristallite erfolgt unabhängig von der Orientierung der Schmelzprismen senkrecht zum Substrat, sodass bei entsprechender Orientierung der Schmelzprismen die Längsorientierung der künstlich aufgewachsenen Kristalle weitgehend identisch mit den Kristallen in den Prismen verläuft. Die Größenordnung von Schmelzkristallen und aufgewachsenem Fluorapatit ist gleich. Innerhalb der Schichten kann eine dichte und gleichmäßige Packung beobachtet werden. Weiterhin weist die aufgebrachte Apatitschicht eine Vickers-Härte auf, die der vom nativen Zahnschmelz entspricht. Die erfindungsgemäß aufgebrachten Apatitschichten weisen insbesondere eine Vickers-Härte im Bereich von 250 bis 400 HV auf.

Mit dem erfindungsgemäßen Kit ist es möglich, Apatitschichten in beliebiger Dicke aufzubringen, da die erreichte Schichtdicke von der Häufigkeit des Gelwechsels abhängig ist. Unter Verwendung des efindungsgemäßen Kits können Wachstumsgeschwindigkeiten von 1 bis 5 µm/Tag, insbesondere von 3 bis 5 µm/Tag erreicht werden.

In einer bevorzugten Ausführungsform wird als erstes Gel ein Gelatine-Glycerin-Gel eingesetzt. Das Gewichtsverhältnis von Gelatine zu Glycerin beträgt dabei bevorzugt 1:5 bis 5:1, insbesondere 1:2 bis 2:1. Glycerin hat die Wirkung, dass der Erweichungspunkt des Gels über die normale menschliche Körpertemperatur angehoben wird. Die erzielte Gelfestigkeit ist notwendig, um das Zweischichtsystem während der Mineralisation zu erhalten, sodass eine gezielte, kontrollierte Kristallabscheidung ermöglicht wird. In einem flüssigen Gel würde es zu einer spontanen Präzipitation feinkristallinen Materials kommen, welches nicht am Zahn anwächst.

Das erste Gel enthält bevorzugt weiterhin Fluoridionen. Das Fluorid kann beispielsweise als Natriumfluorid oder Ammoniumfluorid zugegeben werden. In dieser Ausführungsform kann Fluor-reicher Apatit oder Fluorapatit auf der Oberfläche des Zahnmaterials aufgewachsen werden. Fluorapatit ist insbesondere säureresistenter als der Carbonat-haltige Hydroxyapatit des natürlichen Zahnschmelzes, wobei die Morphologie der sich bildenden Schichten aus Fluorapatit dennoch eine große Ähnlichkeit mit nativem Zahnschmelz aufweist.

Die Wachstumsgeschwindigkeit des Apatits oder Fluorapatits wird unter anderem durch den pH-Wert des ersten Gels bestimmt. Das erste Gel weist einen pH-Wert von 2,0 bis 6,0, insbesondere von 4,0 bis 6,0, mehr bevorzugt von 5,0 bis 5,5 auf.

Bei Verwendung des erfindungsgemäßen Kits wird als zweites Gel ein Schutzgel oder ein Calciumionen enthaltendes Gel eingesetzt. Mit diesem Gel wird die Phosphationen-haltige erste Gelschicht abgedeckt. Durch Verwendung dieser Gelschicht findet überraschenderweise die Apatitbildung ausschließlich an der Oberfläche des Zahnmaterials statt und es kommt nicht zu einer spontanen Auskristallisierung von Apatitkristalliten oder Kompositaggregaten, wie sie bei den im Stand der Technik bekannten Vorgehensweisen beobachtet wird. Im Gegensatz zu den Untersuchungen mit Doppeldiffusionskammern kann somit gezielt eine Beschichtung von Zahnmaterialoberflächen erhalten werden. Während das zweite Gel vorzugsweise keine Materialien enthält, die in den Apatit eingebaut werden sollen, also insbesondere Phosphationen-, Calciumionen- und/oder Fluorionen-frei ist, ist es in bestimmten Ausführungsformen möglich, ein Calciumionen enthaltendes Gel als zweites Gel aufzubringen. Auch damit wurden Wachstumsgeschwindigkeiten von mehreren µm pro Tag erzielt. Zur Bildung des zweiten Gels kann ebenfalls Gelatine eingesetzt werden, wobei ein Gelatine-Glycerin-Gel bevorzugt ist. Als zweites Gel kann aber auch ein anderes Gel, z.B. ausgewählt aus Polysacchariden, etwa Agarose oder Carragnan, sowie Carboxymethylcellulose verwendet werden.

Bei Verwendung des erfindungsgemäßen Kits kann schließlich das mit erstem Gel und gegebenenfalls mit Schutzgel beschichtete Zahnmaterial mit einem Calcium enthaltenden Medium behandelt werden. Als Calcium enthaltendes Medium kann beispielsweise eine Calciumionen enthaltende Lösung oder/und ein Calciumionen enthaltendes Gel eingesetzt werden. Das Calciumionen enthaltende Medium wird dabei bevorzugt unter Verwendung eines wasserlöslichen Calciumionen enthaltenden Salzes, beispielsweise aus CaCl₂ hergestellt.

Das Calciumionen enthaltende Medium weist bevorzugt einen pH-Wert von 6 bis 8 auf.

Mit dem erfindungsgemäßen Kit werden somit die beiden Bestandteile von Apatit, nämlich Phosphationen und Calciumionen jeweils getrennt als eigene Komponente zugeführt, wobei die Calciumphosphatbildung auf der Zahnmaterialoberfläche stattfindet.

Um ein lokales Übersäuern an der Mineralisationsfront durch die Protonenabgabe bei der Apatitbildung zu verhindern, wird das Phosphatgel bevorzugt mit einem Puffersystem versetzt, bevorzugt einem Essigsäurepuffer oder α-α-αTris-(hydroxymethyl)methylamin-Puffer.

Vor der Behandlung mit dem alkalischen Medium oder/und mit dem ersten Gel kann das Zahnmaterial vorbehandelt werden, insbesondere entfettet, angeätzt oder/und gespült werden. Beispielsweise kann für eine bessere Wirksamkeit die Zahnoberfläche zunächst mit Ethanol entfettet und mit Phoshorsäure angeätzt und anschließend mit entionisiertem Wasser gespült werden.

Das erfindungsgemäße Kit eignet sich insbesondere zur Behandlung von Humanzähnen oder Zahnschmelz. Dabei können kariöse Defekte durch Remineralisation behandelt werden oder aber auch das Zahnmaterial prophylaktisch mit einer schützenden Apatit- oder Fluorapatitschicht bedeckt werden. Die Apatitschichten bilden sich sowohl auf Zahnschmelz als auch auf Dentin als Substrat.

Die Erfindung wird durch die folgenden Beispiele weiter erläutert.

### Beispiele

### Beispiel 1 (Vergleichsbeispiel)

### Schritt 1. Vorbereitung des Zahnmaterials

Ein humaner Zahn (beliebig) wurde von seiner Wurzel getrennt und die Krone in etwa 0,5 mm breite Scheiben gesägt. Die Scheiben wurden 30 s lang in einer 30 %-igen Phosphorsäurelösung getaucht, mit entionsiertem Wasser gewaschen und getrocknet.

### Schritt 2. Vorbereitung des Gels

Aus 8,56 g Gelatine, 8,24 g 85 %-iger Glycerinlösung, 7,26 g H₂O, 1,8 ml 2N NaOH, 2,7 ml 2 N HAc, 13,8 mg NaF, 236 mg Na₂HPO₄ wurde bei 80 °C unter Rühren ein homogenes Gel hergestellt, dessen pH-Wert bei 5,0 lag. Ein weiteres Gel wurde aus 8,56 g Gelatine, 8,24 g 85 %-iger Glycerinlösung und 11,76 g H₂O hergestellt. Eine 0,133 molare Calciumlösung wurde aus Cacl₂-Salz hergestellt.

### Schritt 3. Induzierte Mineralisation an der Zahnoberfläche

Die Oberfläche der Zahnscheiben wurde mit etwa 0,5 ml des phoshathaltigen Gels bestrichen. Nach dessen Verfestigung erfolgte eine Bedeckung mit etwa 0,5 ml des zusatzfreien Gels. Die Zahnscheibe wurde in ein einseitig verschlossenes Plastikrohr eingefügt und in einer Calciumlösung bei 37 °C gelagert. Das Gel und die Lösung wurden alle 7 Tage erneuert, insgesamt 16 x. Zur Begutachtung der aufgewachsenen Schicht erfolgte ein Aufbruch der Probe senkrecht zur Schnittfläche, um die Schichtdicke ausmessen zu können. Es bildete sich eine gleichmäßige Schicht elongierter Kristallite mit einer Schichtdicke von 7,2 µm. Das entspricht einer Wachstumsgeschwindigkeit von etwa 450 nm/Woche.

### Beispiel 2

Die Vorgehensweise erfolgt analog Beispiel 1, jedoch wurde zunächst eine Behandlung mit einer alkalischen Lösung durchgeführt. Weiterhin erfolgte die Lagerung in Calciumlösung, wie oben beschrieben, oder in SBF (Simulated Body Fluid mit 142 mM Na⁺, 5 mM K⁺, 15 mM Mg²⁺, 25 mM Ca²⁺, 148,8 mM Cl-, 4,2 mM HCO₃²⁻, 1 mM HPO₄²⁻, 0,5 mM SO₄²⁻) oder trocken. Dabei konnten Wachstumsgeschwindigkeiten von 0,9 bis 1,4 µm/Tag bei trockener Lagerung, von 1,4 bis 1,9 µm/Tag bei Lagerung in SWF und von 2,1 µm/Tag bei Lagerung in einer Ca-Lösung beobachtet werden. Die Schichtbildung erfolgte bevorzugt auf Dentin.

### Beispiel 3

Das Vorgehen entspricht Beispiel 2. Es wurden jedoch Fluorapatit-Sphärolithen mit einem Durchmesser von ≤ 20 µm, einem Durchmesser von 20 bis 25 µm bzw. einem Durchmesser von 50 bis 100 µm dem ersten Gel zugesetzt. Dabei konnten Wachstumsgeschwindigkeiten von 5 µm/Tag bei Zusatz von Fluorapatit-Sphärolithen mit einem Durchmesser ≤ 20 µm, eine Wachstumsgeschwindigkeit von 4 µm/Tag bei Zusatz von Fluorapatit-Sphärolithen mit einem Durchmesser von 20 bis 25 µm und eine Wachstumsgeschwindigkeit von 1,25 µm/Tag bei Zusatz von Fluorapatit-Sphärolithen mit einem Durchmesser von 50 bis 100 µm erhalten werden.

### Beispiel 4

Das Vorgehen entspricht Beispiel 2, wobei anstelle der Calciumlösung ein Calciumgel eingesetzt wurde. Auch hier konnten vergleichbare Wachstumsraten festgestellt werden.

### Beispiel 5

Das Vorgehen entspricht Beispiel 4, wobei das Schutzgel weggelassen wurde. Auch hier wurden Wachstumsraten von 1,8 bis 5 µm/Tag erhalten.

## Patentansprüche

1. Kit, umfassend
(i) ein alkalisches Medium zur Vorbehandlung eines Zahnmaterials,
(ii) ein erstes Gel, welches Gelatine sowie Phosphationen umfasst, wobei die Erweichungstemperatur des ersten Gels Im Bereich von 38 bis 45 °C liegt und wobei das erste Gel einen pH-Wert von 2,0 bis 6,0 aufweist, zum Auftragen auf das mit dem alkalischen Medium vorbehandelte Zahnmaterial, sowie
(iii) ein zweites Gel, welches frei von Phosphationen Ist, zum Abdecken der ersten Gelschicht, wobei die Erweichungstemperatur des zweiten Gels im Bereich von 38 bis 45 °C liegt, und
(iva) ein Calciumionen enthaltendes Medium, zum Auftragen auf die zweite Gelschicht
oder
(ivb) ein Calciumionen enthaltendes zweites Gel zum Abdecken der ersten Gelschicht anstelle des Gels (iii), wobei die Erweichungstemperatur des zweiten Gels Im Bereich von 38 bis 45 °C liegt.

2. Kit nach Anspruch 1,
**dadurch gekennzeichnet,**
**dass** das alkalische Medium eine alkalische Lösung oder ein alkalisches Gel ist.

3. Kit nach Anspruch 1 oder 2,
**dadurch gekennzeichnet,**
**dass** das alkalische Medium einen pH-Wert von 7,1 bis 14 aufweist.

4. Kit nach Anspruch 3,
**dadurch gekennzeichnet,**
**dass** das alkalische Medium einen pH-Wert von 7,5 bis 10 aufweist.

5. Kit nach Anspruch 3,
**dadurch gekennzeichnet,**
**dass** das alkalische Medium einen pH-Wert von 8 bis 9 aufweist.

6. Kit nach einem der vorhergehenden Ansprüche,
**dadurch gekennzeichnet,**
**dass** das alkalische Medium 0,05 bis 1N NaOH ist.

7. Kit nach einem der vorhergehenden Ansprüche,
**dadurch gekennzeichnet,**
**dass** das alkalische Medium weiterhin Calciumlonen enthält.

8. Kit nach einem der vorhergehenden Ansprüche,
**dadurch gekennzeichnet,**
**dass** das erste Gel ein Gelatine-Glycerin-Gel ist.

9. Kit nach einem der vorhergehenden Ansprüche,
**dadurch gekennzeichnet,**
**dass** das erste Gel weiterhin Fluoridionen enthält.

10. Kit nach einem der vorhergehenden Ansprüche,
**dadurch gekennzeichnet,**
**dass** das erste Gel weiterhin mindestens eine Calciumphosphatverbindung enthält.

11. Kit nach Anspruch 10,
**dadurch gekennzeichnet,**
**dass** das erste Gel weiterhin eine Calciumphosphatverbindung enthält, ausgewählt aus Fluorapatit, Monetit, Brushit, amorphem Calciumphosphat und Hydroxylapatit.

12. Kit nach einem der Ansprüche 10 oder 11,
**dadurch gekennzeichnet,**
**dass** das erste Gel 5 bis 30 Gew.-% an Calciumphosphatverbindungen enthält.

13. Kit nach einem der Ansprüche 10 bis 12,
**dadurch gekennzeichnet,**
**dass** das erste Gel sphärische Partikel von Calciumphosphatverbindungen enthält.

14. Kit nach einem der Ansprüche 10 bis 13,
**dadurch gekennzeichnet,**
**dass** die Calciumphosphatverbindung in Form von Partikeln mit einer mittleren Größe von 5 bis 50 µm vorliegt.

15. Kit nach Anspruch 14,
**dadurch gekennzeichnet,**
**dass** die Calciumphosphatverbindung in Form von Partikeln mit einer mittleren Größe von 10 bis 20 µm vorliegt.

16. Kit nach einem der Ansprüche 10 bis 14,
**dadurch gekennzeichnet,**
**dass** die Calciumphosphatverbindung Fluorapatit ist.

17. Kit nach einem der vorhergehenden Ansprüche,
**dadurch gekennzeichnet,**
**dass** das zweite Gel fluoridionenfrei ist.

18. Kit nach einem der vorhergehenden Ansprüche,
**dadurch gekennzeichnet,**
**dass** das zweite Gel ausgewählt wird aus Gelatine-Glycerin-Gelen, Polysaccharid-Gelen oder Carboxymethylcellulose-Gelen.

19. Kit nach einem der vorhergehenden Ansprüche,
**dadurch gekennzeichnet,**
**dass** das Calciumionen enthaltende Medium eine Calciumlonen enthaltende Lösung oder ein Calciumionen enthaltendes Gel ist.

20. Kit nach einem der vorhergehenden Ansprüche,
**dadurch gekennzeichnet,**
**dass** das Calciumionen enthaltende Medium einen pH-Wort von 6 bis 8 aufweist.

21. Kit nach einem der vorhergehenden Ansprüche zum Aufwachsen von Apatit oder/und Dentin auf Zahnmaterial.

## Claims

1. Kit comprising:
(i) an alkaline medium for pretreating tooth material,
(ii) a first gel, which comprises gelatin and phosphate ions, wherein the softening temperature of the first gel is in the range of from 38 to 45 °C and wherein the first gel has a pH value of from 2.0 to 6.0, for application to the tooth material which has been pretreated with the alkaline medium, and
(iii) a second gel, which is free of phosphate ions, for covering the first layer of gel, wherein the softening temperature of the second gel is in the range of from 38 to 45 °C, and
(iva) a medium, containing calcium ions, for application to the second layer of gel, or
(ivb) a second gel, containing calcium ions, for covering the first layer of gel instead of the gel (iii), wherein the softening temperature of the second gel is in the range of from 38 to 45 °C.

2. Kit according to claim 1, **characterised in that** the alkaline medium is an alkaline solution or an alkaline gel.

3. Kit according to either claim 1 or claim 2, **characterised in that** the alkaline medium has a pH value of from 7.1 to 14.

4. Kit according to claim 3, **characterised in that** the alkaline medium has a pH value of from 7.5 to 10.

5. Kit according to claim 3, **characterised in that** the alkaline medium has a pH value of from 8 to 9.

6. Kit according to any of the preceding claims, **characterised in that** the alkaline medium is 0.05 to 1 N NaOH.

7. Kit according to any of the preceding claims, **characterised in that** the alkaline medium furthermore contains calcium ions.

8. Kit according to any of the preceding claims, **characterised in that** the first gel is a gelatin-glycerol gel.

9. Kit according to any of the preceding claims, **characterised in that** the first gel furthermore contains fluoride ions.

10. Kit according to any of the preceding claims, **characterised in that** the first gel further comprises at least one calcium phosphate compound.

11. Kit according to claim 10, **characterised in that** the first gel further comprises a calcium phosphate compound selected from fluorapatite, monetite, brushite, amorphous calcium phosphate and hydroxylapatite.

12. Kit according to either claim 10 or 11, **characterised in that** the first gel contains from 5 to 30 wt.% of calcium phosphate compounds.

13. Kit according to any of claims 10 to 12, **characterised in that** the first gel contains spherical particles of calcium phosphate compounds.

14. Kit according to any of claims 10 to 13, **characterised in that** the calcium phosphate compound is present in the form of particles having an average size of from 5 to 50 µm.

15. Kit according to claim 14, **characterised in that** the calcium phosphate compound is present in the form of particles having an average size of from 10 to 20 µm.

16. Kit according to any of claims 10 to 14, **characterised in that** the calcium phosphate compound is fluorapatite.

17. Kit according to any of the preceding claims, **characterised in that** the second gel is free of fluoride ions.

18. Kit according to any of the preceding claims, **characterised in that** the second gel is selected from gelatin-glycerol gels, polysaccharide gels or carboxymethyl-cellulose gels.

19. Kit according to any of the preceding claims, **characterised in that** the medium containing calcium ions is a solution containing calcium ions or a gel containing calcium ions.

20. Kit according to any of the preceding claims, **characterised in that** the medium containing calcium ions has a pH value of from 6 to 8.

21. Kit according to any of the preceding claims for growing apatite and/or dentine on tooth material.

## Revendications

1. Kit, comprenant
(i) un milieu alcalin destiné au prétraitement d'une matière dentaire,
(ii) un premier gel qui comprend de la gélatine ainsi que des ions phosphate, la température de ramollissement du premier gel se situant dans la plage de 38 à 45 °C, et le premier gel présentant un pH de 2,0 à 6,0, destiné à être appliqué sur la matière dentaire prétraitée avec le milieu alcalin, ainsi
(iii) qu'un deuxième gel, qui est exempt d'ions phosphate, destiné à recouvrir la première couche de gel, la température de ramollissement du deuxième gel se situant dans la plage de 38 à 45 °C, et
(iva) un milieu contenant des ions calcium, destiné être à appliqué sur la deuxième couche de gel
ou
(ivb) un deuxième gel contenant des ions calcium destiné à recouvrir la première couche de gel à la place du gel (iii), la température de ramollissement du deuxième gel se situant dans la plage de 38 à 45 °C.

2. Kit selon la revendication 1,
**caractérisé en ce que**
le milieu alcalin est une solution alcaline ou un gel alcalin.

3. Kit selon la revendication 1 ou 2,
**caractérisé en ce que**
le milieu alcalin présente un pH de 7,1 à 14.

4. Kit selon la revendication 3,
**caractérisé en ce que**
le milieu alcalin présente un pH de 7,5 à 10.

5. Kit selon la revendication 3,
**caractérisé en ce que**
le milieu alcalin présente un pH de 8 à 9.

6. Kit selon l'une des revendications précédentes,
**caractérisé en ce que**
le milieu alcalin est 0,05 à 1 N NaOH.

7. Kit selon l'une des revendications précédentes,
**caractérisé en ce que**
le milieu alcalin contient également des ions calcium.

8. Kit selon l'une des revendications précédentes,
**caractérisé en ce que**
le premier gel est un gel gélatine-glycérine.

9. Kit selon l'une des revendications précédentes,
**caractérisé en ce que**
le premier gel contient également des ions fluorure.

10. Kit selon l'une des revendications précédentes,
**caractérisé en ce que**
le premier gel contient également au moins un composé de phosphate de calcium.

11. Kit selon la revendication 10,
**caractérisé en ce que**
le premier gel contient également un composé de phosphate de calcium sélectionné parmi la fluoroapatite, la monétite, la brushite, le phosphate de calcium amorphe et l'hydroxyapatite.

12. Kit selon l'une des revendications 10 ou 11,
**caractérisé en ce que**
le premier gel contient 5 à 30 % en poids de composés de phosphate de calcium.

13. Kit selon l'une des revendications 10 à 12,
**caractérisé en ce que**
le premier gel contient des particules sphériques de composés de phosphate de calcium.

14. Kit selon l'une des revendications 10 à 13,
**caractérisé en ce que**
le composé de phosphate de calcium se présente sous la forme de particules avec une taille moyenne de 5 à 50 µm.

15. Kit selon la revendication 14,
**caractérisé en ce que**
le composé de phosphate de calcium se présente sous la forme de particules avec une taille moyenne de 10 à 20 µm.

16. Kit selon l'une des revendications 10 à 14,
**caractérisé en ce que**
le composé de phosphate de calcium est de la fluoroapatite.

17. Kit selon l'une des revendications précédentes,
**caractérisé en ce que**
le deuxième gel est exempt d'ions fluorure.

18. Kit selon l'une des revendications précédentes,
**caractérisé en ce que**
le deuxième gel est sélectionné parmi des gels de gélatine-glycérine, des gels de polysaccharide ou des gels de carboxyméthylcellulose.

19. Kit selon l'une des revendications précédentes,
**caractérisé en ce que**
le milieu contenant des ions calcium est une solution contenant des ions calcium ou un gel contenant des ions calcium.

20. Kit selon l'une des revendications précédentes,
**caractérisé en ce que**
le milieu contenant des ions calcium présente un pH de 6 à 8.

21. Kit selon l'une des revendications précédentes, destiné à la croissance d'apatite et/ou de dentine sur de la matière dentaire.
